(19) Europäisches Patentamt / European Patent Office / Office européen des brevets

(11) **EP 4 310 069 A1**

(12) **EUROPEAN PATENT APPLICATION**
published in accordance with Art. 153(4) EPC

(43) Date of publication:
**24.01.2024 Bulletin 2024/04**

(21) Application number: 22771535.6

(22) Date of filing: **18.03.2022**

(51) International Patent Classification (IPC):
*C07C 51/235* [(2006.01)]    *C07C 59/125* [(2006.01)]
*C07B 61/00* [(2006.01)]    *B01J 37/04* [(2006.01)]
*B01J 23/644* [(2006.01)]

(52) Cooperative Patent Classification (CPC):
**B01J 23/644; B01J 37/04; C07B 61/00;**
**C07C 51/235; C07C 59/125**

(86) International application number:
**PCT/JP2022/012491**

(87) International publication number:
**WO 2022/196790 (22.09.2022 Gazette 2022/38)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **19.03.2021   JP 2021046220**

(71) Applicant: **Kao Corporation**
**Chuo-ku**
**Tokyo 103-8210 (JP)**

(72) Inventor: **NOMURA, Yukiko**
**Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

(54) **METHOD FOR PRODUCING OXIDE AND METHOD FOR PRODUCING PT/BI COMPOSITE CATALYST**

(57)    The invention relates to a production method for an oxide capable of efficiently producing an oxide of an organic compound in the presence of raw materials for a Pt/Bi composite catalyst, and to a production method for a Pt/Bi composite catalyst capable of producing a catalyst that exhibits a high activity for dehydrogenation oxidation reaction of an organic compound even in the presence of an organic compound to be a raw material for an oxide. The oxide production method includes subjecting an organic compound having one primary hydroxy group to a dehydrogenative oxidation reaction in the presence of Pt supported by a carrier, a Bi ion source and water, under the condition such that the minimum value of the pH during the reaction is less than 7, thereby obtaining an oxide of the organic compound.

EP 4 310 069 A1

**Description**

Field of the Invention

The present invention relates to a method for producing an oxide, and to a method for producing a Pt/Bi composite catalyst.

Background of the Invention

Heretofore there is known a method of catalytic dehydrogenative oxidation of a hydroxy compound or an aldehyde compound to convert into the corresponding carboxy compound or a ketone compound, using a noble metal catalyst Pt as combined with a promoter Bi.

JP2016-120484A (PTL 1) discloses a method for preparing a catalyst by adding an aqueous dispersion of a catalyst in which Pt is supported by an active carbon to an aqueous solution containing Bi in a state of an ion, and a method for producing an oxide by dehydrogenative oxidation of an alcohol or the like in the presence of a catalyst obtained in the preparation method.

JPH01-146840A (PTL 2) discloses a method for producing an ether-carboxylic acid by oxidizing a polyalkoxyalcohol or an aliphatic alcohol alkoxylate in an alkaline aqueous phase in the presence of a catalyst containing a noble metal of the Group VIII of the Periodic Table, such as Pd or Pt.

Summary of the Invention

The present invention relates to a method for producing an oxide by subjecting an organic compound having one primary hydroxy group to a dehydrogenative oxidation reaction in the presence of Pt supported by a carrier, a Bi ion source and water under the condition such that the minimum value of pH during reaction is less than 7, thereby to obtain an oxide of the organic compound.

Also the present invention relates to a method for producing a Pt/Bi composite catalyst by mixing a Pt supported by a carrier and a Bi ion source and reacting them under the condition such that the minimum value of pH during reaction is less than 7, in the presence of an organic compound having one primary hydroxy group and water.

The catalyst preparation method described in PTL 1 is a process different from the production process of an oxide of an alcohol and the like, and therefore a catalyst production equipment is needed separately from the production equipment for an oxide of an alcohol and the like. In addition, the catalyst preparation process needs a step of separating and purifying the catalyst apart from the step of preparing a raw material for the catalyst, and further, the prepared catalyst needs to be stored under appropriate conditions before use for production of an oxide of an alcohol. Accordingly, the method has a problem of requiring much cost and labor for catalyst preparation apart from production of an oxide of an alcohol. In addition, the ether-carboxylic acid production method described in PTL 2 has a problem of low productivity of ether-carboxylic acid.

The present invention relates to an oxide production method capable of efficiently obtaining an oxide of an organic compound in the presence of a raw material for a Pt/Bi composite catalyst, and to a production method for a Pt/Bi composite catalyst capable of producing a catalyst having a high activity for dehydrogenative oxidation of an organic compound, even in the presence of an organic compound that is to be a raw material for an oxide.

The present inventors have found that an oxide of an organic compound can be effectively obtained even in the presence of a raw material for a Pt/Bi composite catalyst by controlling the pH during the reaction to be less than 7, and that a catalyst exhibiting a high activity for dehydrogenative oxidation reaction of an organic compound can be produced by controlling the pH during the reaction to be less than 7, even in the presence of an organic compound that is to be a raw material for an oxide.

The present invention relates to the following [1] and [2].

[1] A production method for an oxide, including subjecting an organic compound having one primary hydroxy group to a dehydrogenative oxidation reaction in the presence of Pt supported by a carrier, a Bi ion source and water under the condition such that the minimum value of the pH during the reaction is less than 7, thereby obtaining an oxide of the organic compound.

[2] A production method for a Pt/Bi composite catalyst, including mixing Pt supported by a carrier and Bi ion source in the presence of an organic compound having one primary hydroxy group and water and reacting them under the condition such that the minimum value of the pH during the reaction is less than 7.

According to the present invention, there can be provided a production method for an oxide capable of efficiently obtaining an oxide of an organic compound in the presence of a raw material for a Pt/Bi composite catalyst, and a production method for a Pt/Bi composite catalyst capable of producing a catalyst that exhibits a high activity for dehydrogenative oxidation of an organic compound even in the presence of an organic compound that is to be a raw material for an oxide.

Hereinunder the present invention is described in detail.

[Production Method for Oxide]

The production method for an oxide of the present invention is an oxide production method for obtaining an oxide of an organic compound, that includes subjecting an organic compound having one primary hydroxy group (hereinunder this may be simply referred to as an "organic compound") to a dehydrogenative oxidation reaction in the presence of Pt supported by a carrier, a Bi ion source and water under the condition such that the minimum value of the pH during the reaction is less than 7, thereby obtaining an oxide of the organic compound.

According to the production method for an oxide of the present invention, an oxide of an organic compound can be efficiently obtained.

Though not clear, the reason why the above-mentioned effect can be attained is presumed as follows.

In the oxide production method of the present invention, the Pt/Bi composite catalyst to be used for the reaction is, in the form of a raw material, charged together in carrying out a dehydrogenative oxidation reaction of an organic compound to thereby subjecting the organic compound to a dehydrogenative oxidation reaction while the Pt/Bi composite catalyst is produced in the reaction system.

In the case of preparing a catalyst such as a Pt/Bi composite catalyst heretofore used in dehydrogenative oxidation reaction of an organic compound, in general, a Pt ion source and a Bi ion source are mixed in an alkaline condition by adding a reducing agent thereto for precipitation of Pt and Bi thereby preparing a composite catalyst of Pt and Bi, but in that case, Bi may segregate, and therefore it is considered that the amount of the active structure effective for a dehydrogenative oxidation reaction of an organic compound is small and the catalyst activity may be thereby low.

On the other hand, though the reason is not clear, it is considered that, in the present invention, when Pt supported by a carrier and a Bi ion source are brought into contact with each other under the condition such that the minimum value of the pH during the reaction is less than 7, Bi can be uniformly reductively precipitated on Pt even in the absence of a reducing agent, and accordingly, much active structure effective for a dehydrogenative oxidation reaction of an organic compound can be formed and the activity of the resultant catalyst can be high.

Heretofore, in the case of a dehydrogenative oxidation reaction of an organic compound having a hydroxy group or an aldehyde group where the catalyst activity is low, the reaction system needs to be under an alkaline condition from the viewpoint of increasing the reactivity, but in the oxide production method of the present invention, the minimum value of the pH during the reaction is less than 7 and the catalyst activity can be increased as mentioned above, and therefore the productivity of an oxide of an organic compound can be increased without addition of an alkaline agent.

Further, in the case of a conventional dehydrogenative oxidation reaction under an alkaline condition as mentioned above, an alkaline agent is added to make the system alkaline condition, and therefore in the case, a neutralized salt of an oxide is formed as a by-product. In the case where a neutralized salt of an oxide is formed in the reaction system and where the ratio of oxide/water is high as compared with an oxide alone, the viscosity of the reaction liquid increases so that there occurs a phenomenon of gelation of the reaction liquid. For these reasons, in oxidation under a conventional alkaline condition, the reaction is inevitably carried out in the condition having a low active ingredient concentration such that the ratio of oxide/water is lowered, and accordingly, it is difficult to increase the productivity. On the other hand, in the oxide production method of the present invention, formation of a neutralized salt of an oxide as a by-product can be suppressed, and viscosity increase of the reaction liquid and gelation thereof can be suppressed, and accordingly, an organic compound can be effectively subjected to a dehydrogenative oxidation reaction in a composition having a high effective ingredient concentration where the ratio of oxide/water is increased, and the productivity can be thereby enhanced. Further, generation of a salt to form in taking out the oxide from the neutralized salt of an oxide formed as a by-product can be suppressed, and accordingly, generation of a waste liquid can be reduced and the environmental load can be thereby reduced.

In the oxide production method of the present invention, from the viewpoint of producing an oxide of an organic compound at a high productivity, preferably the method is accompanied by preparation of a Pt/Bi composite catalyst.

Though not clear, the reason why the method is preferably accompanied by preparation of a Pt/Bi composite catalyst can be considered as follows.

By mixing an organic compound in the presence of the Pt supported by a carrier and the Bi ion source mentioned above, the organic compound undergoes dehydrogenative oxidation reaction. Accordingly, a small amount of an oxide of the organic compound is formed to give an oxide-containing solution, and therefore, it is presumed that the Bi ion source dissolves to generate a Bi ion and Bi is reductively precipitated on Pt to produce a Pt/Bi composite catalyst having a higher activity than a Pt catalyst, in the system. In addition, it is presumed that, along with production of the Pt/Bi composite catalyst, the dehydrogenative oxidation of the organic compound is promoted and an oxide of the organic compound can be obtained at a high yield.

From the above, in the oxide production method of the present invention, a catalyst can be prepared simultaneously in the production step of an oxide, and therefore the oxide production method of the present invention does not require any additional equipment and process for separately producing the catalyst, and an oxide of an organic compound can be efficiently produced therefore providing great advantages of production efficiency and production cost.

In the present description, unless otherwise specifically indicated, Pt means a platinum, Bi means a bismuth, and C means an active carbon. Also in the present description, unless otherwise specifically indicated, "%" is "% by mass".

<Organic Compound having one primary hydroxy group>

The production method for an oxide of the present invention is performed by dehydrogenative oxidation of an organic compound having one primary hydroxy group (hereinunder this may be simply referred to as an organic compound).

The organic compound having one primary hydroxy group includes an aliphatic alcohol, a polyoxyalkylene alkyl ether, an amide alcohol, and an aromatic alcohol. From the viewpoint of high hydrophilicity, preferred is at least one selected from an aliphatic alcohol, a polyoxyalkylene alkyl ether and an amide alcohol, and more preferred is at least one selected from an aliphatic alcohol and a polyoxyalkylene alkyl ether.

The aliphatic alcohol or the polyoxyalkylene alkyl ether is preferably one or two or more represented by the following general formula (1) or general formula (2).

$$R^1O\text{-}H \qquad (1)$$

In the general formula (1), $R^1$ represents a monovalent aliphatic hydrocarbon group having 2 or more and 40 or less carbon atoms.

$$R^2O\text{-}(AO)_n\text{-}H \qquad (2)$$

In the formula (2), $R^2$ represents a monovalent aliphatic hydrocarbon group having 2 or more and 40 or less carbon atoms, A represents an alkanediyl group having 2 or more and 4 or less carbon atoms, AO represents an alkyleneoxy group, n represents an average value of an addition molar number of the alkyleneoxy group, and is 1 or more and 30 or less.

$R^1$ is, from the viewpoint of reactivity, preferably a linear or branched, primary monovalent aliphatic hydrocarbon group, more preferably a linear or branched, primary alkyl or alkenyl group, even more preferably a linear primary alkyl group.

Though not specifically limited, the carbon number of $R^1$ can be 6 or more, 8 or more, 10 or more, or 12 or more, and is, from the viewpoint of reactivity, preferably 36 or less, more preferably 22 or less, even more preferably 18 or less, further more preferably 14 or less.

$R^2$ is, from the viewpoint of reactivity, preferably a linear or branched, primary aliphatic hydrocarbon group, more preferably a linear or branched, primary alkyl or alkenyl group, even more preferably a linear primary alkyl group.

Though not specifically limited, the carbon number of $R^2$ can be 6 or more, 8 or more, 10 or more, or 12 or more, and is, from the viewpoint of reactivity, preferably 36 or less, more preferably 22 or less, even more preferably 18 or less, further more preferably 14 or less.

A is, from the viewpoint of reactivity, preferably an ethylene group or a propylene group, more preferably an ethylene group.

Similarly, AO is, from the viewpoint of reactivity, preferably an ethyleneoxy group (EO) or a propyleneoxy group (PO), more preferably an ethyleneoxy group(EO).

n is, from the viewpoint of reactivity, preferably 3 or more, and is preferably 25 or less, more preferably 20 or less, even more preferably 16 or less, further more preferably 12 or less.

For the oxide production method of the present invention, the intensity of hydrophilic of the organic compound has an influence on the reactivity, and a higher hydrophilicity brings about higher reactivity between the Pt/Bi composite catalyst and the organic compound. Specifically, a higher IOB value (inorganic/organic balance value) IV/OV that is calculated from the IV (inorganic value) and the OV (organic value) on an organic conceptual diagram of an organic compound brings about a higher reactivity.

Accordingly, an organic compound is, from the viewpoint of the intensity of hydrophilicity, preferably a polyoxyalkylene alkyl ether, more preferably a polyoxyalkylene alkyl ether represented by the general formula (2).

The IOB value based on the organic conceptual diagram of the organic compound is, from the viewpoint of reactivity, preferably 0.3 or more, more preferably 1 or more, even more preferably 4 or more. The IOB value of the organic compound can be less than 5.

The oxide production method of the present invention includes subjecting the above-mentioned organic compound to a dehydrogenative oxidation reaction to obtain a carboxylic acid compound or a salt of a carboxylic acid compound as an oxide.

The carboxylic acid compound or the salt of a carboxylic acid compound includes a carboxylic acid compound or a salt of a carboxylic acid compound obtained by subjecting the above-mentioned aliphatic alcohol or polyoxyalkylene alkyl ether to a dehydrogenating oxidation reaction, for example, including a so-called ether carboxylate or a salt thereof obtained by subjecting a polyoxyalkylene alkyl ether to an oxidation reaction.

<Pt supported by carrier>

The carrier for use for Pt supported by a carrier is, from the viewpoint of reaction under the condition of pH of less than 7, preferably a carrier selected from titania, zirconia and active carbon, more preferably active carbon.

Not specifically limited, the active carbon can be any one capable of adsorbing and supporting Pt, and any kind of active carbon is employable here. Examples of active carbon include a vegetable active carbon such as active carbon from coconut shell, a mineral active carbon such as active carbon from coal, and active carbon of pulp waste liquid, synthetic resin and organic waste matter. The activation method, the pore size distribution and the shape of active carbon are not specifically limited. However, active carbon may differ in reactivity. As compared with vegetable active carbon, active carbon from coal is preferred as having high activity. On the other hand, regarding the strength enough not to crack in filtration, vegetable active carbon is preferred as compared with active carbon from coal.

The particle size of Pt supported by a carrier is not specifically limited, but is, from the viewpoint of increasing the amount of Pt supported, preferably 1 nm or more, more preferably 3 nm or more, and is, from the viewpoint of increasing the dispersibility of Pt on the surface of a carrier, preferably 20 nm or less, more preferably 15 nm or less, even more preferably 10 nm or less.

The amount of Pt supported in the carrier-supported Pt, that is, relative to the total amount of carrier and Pt is, from the viewpoint of the reactivity of dehydrogenative oxidation reaction of an organic compound, preferably 0.1% by mass or more, more preferably 1% by mass or more, even more preferably 2% by mass or more, further more preferably 3% by mass or more, further more preferably 5% by mass or more, and is, from the viewpoint of enhancing the dispersibility of Pt on the carrier surface, preferably 20% by mass or less, more preferably 15% by mass or less, even more preferably 10% by mass or less. Pt supported by a carrier can be prepared by a known impregnation method or precipitation method, and commercial products thereof can also be used.

The amount to be charged of Pt supported by a carrier can be arbitrarily selected in a range in which a practicable reaction speed can be realized, depending on the reaction temperature or the reaction pressure, and when the reaction is in a batchwise mode, the amount is, from the viewpoint of reactivity, relative to 100 parts by mass of the organic compound, preferably 1 part by mass or more, more preferably 3 parts by mass or more, even more preferably 4 parts by mass or more, and is preferably 10 parts by mass or less, more preferably 8 parts by mass or less, even more preferably 6 parts by mass or less.

<Bi Ion Source>

The Bi ion source is, from the viewpoint of improving the catalyst activity of the produced Pt/Bi composite catalyst and from the viewpoint of efficiently producing an oxide, preferably water-insoluble.

The Bi ion source is preferably at least one selected from bismuth nitrate pentahydrate ($Bi(NO_3)_3 \cdot 5H_2O$), bismuth oxide ($Bi_2O_3$), basic bismuth carbonate (($BiO)_2CO_3$) and bismuth hydroxide ($Bi(OH)_3$).

Depending on the Bi ion source used, the catalyst activity of the resultant Pt/Bi composite catalyst differs, and in the present invention, when bismuth oxide is used as the Bi ion source, a Pt/Bi composite catalyst having high catalytic activity can be obtained, and an oxide of an organic compound can be efficiently obtained. Consequently, bismuth oxide is preferred as the Bi ion source.

The amount to be charged of the Bi ion source is, from the viewpoint of catalyst production efficiency and from the viewpoint of production efficiency of an oxide of an organic compound, preferably 0.01 parts by mass or more relative to 100 parts by mass of the organic compound, more preferably 0.03 parts by mass or more, even more preferably 0.05 parts by mass or more, and is preferably 1 part by mass or less, more preferably 0.5 parts by mass or less, even more preferably 0.3 parts by mass or less.

In the oxide production method of the present invention, the mass ratio (atomic ratio) of Bi to the amount of Pt to be charged, Bi/Pt is, from the viewpoint of improving the productivity of the product in oxidation reaction, preferably 0.05 or more, more preferably 0.1 or more, and is preferably 2.0 or less, more preferably 1.8 or less, even more preferably 1.5 or less, further more preferably 1.4 or less, further more preferably 1.2 or less, further more preferably 1.0 or less.

In the case where bismuth oxide is charge as a Bi ion source, the reactivity to prepare the Pt/Bi composite catalyst differs depending on the amount of bismuth oxide charged. Even when a small amount of bismuth oxide is added, the reactivity to prepare the Pt/Bi composite oxide improves. A preferred embodiment of the amount of bismuth oxide to be charged relative to 100 parts by mass of the organic compound is the same as the preferred embodiment of the amount of Bi ion source to be charged, as mentioned above.

<Water>

The oxide production method of the present invention is performed in the presence of water, and preferred examples of water include ion-exchanged water, distilled water and pure water.

The amount of water to be used is, from the viewpoint of improving the productivity of the oxide of an organic compound and suppressing the increase in the viscosity of the liquid phase, preferably 15 parts by mass or more relative to 100 parts by mass of the organic compound, more preferably 20 parts by mass or more, even more preferably 35 parts by mass or less, further more preferably 30 parts by mass or less.

The amount of water to be used is a total amount of the charged water, the water content in Pt supported by a carrier and the water content of the Bi ion source.

<Reaction Condition>

(pH during reaction)

In the oxide production method of the present invention, the minimum value of the pH during the reaction is less than 7. By controlling the minimum value of the pH during the reaction to less than 7, the reaction system can be made acidic so that the Bi ion source can dissolve to easily form a Bi ion, and it is considered that Bi alone from a Bi ion can be prevented from precipitating on the carrier such as active carbon so as to form a structure having a high activity by reductive precipitation of Bi around Pt, and accordingly, reactivity to produce a highly-active Pt/Bi composite catalyst and reactivity for a dehydrogenating oxidation reaction of an organic compound can be greatly improved.

In the oxide production method of the present invention, an organic compound undergoes a dehydrogenative oxidation reaction to form an oxide, and therefore the minimum value of the pH during the reaction can be controlled to less than 7 without addition of an alkaline agent to the reaction system.

Regarding the pH in the oxide production method of the present invention, when a Bi oxide is used as a Bi ion source, even though the reaction system is alkaline in the stage of charging a raw material, the reaction system changes to acidic with increase in the oxide of an organic compound in the reaction system as the reaction progresses.

The minimum value of the pH at the end of the reaction is, from the viewpoint of improving the production efficiency for the Pt/Bi composite catalyst and from the viewpoint of improving the production efficiency for the oxide of an organic compound, preferably 6 or less, more preferably 5 or less, even more preferably 4 or less, and is preferably 1 or more, more preferably 1.5 or more, even more preferably 2 or more.

(Reaction Temperature)

The reaction temperature in the oxide production method of the present invention is, from the viewpoint of the reactivity in producing the Pt/Bi composite catalyst and the reactivity in a dehydrogenative oxidation reaction of the organic compound, preferably 50°C or higher, more preferably 60°C or higher, and is, from the viewpoint of the equipment load, preferably 100°C or lower, more preferably 90°C or lower, even more preferably 85°C or lower.

(Oxygen-containing Gas)

In the oxide production method of the present invention, from the viewpoint of subjecting the organic compound to a dehydrogenative oxidation reaction, it is preferable to supply oxygen into the reaction system.

Oxygen supply can be attained by making an oxygen-containing gas flow to run through the liquid phase. The oxygen-containing gas includes an oxygen gas and a gas that contains oxygen, such as air.

In the case where an oxygen-containing mixed gas is used, the gas to be combined with oxygen is, from the viewpoint of not having any influence on activity, preferably an inert gas such as helium, argon and nitrogen.

The oxygen concentration in the oxygen-containing gas is, from the viewpoint of the productivity of the oxide, preferably 10% by volume or more, more preferably 50% by volume or more, even more preferably 70% by volume or more, further more preferably 90% by volume or more, further more preferably 100% by volume.

(Reaction Pressure)

In the oxide production method of the present invention, the reaction can be performed under normal pressure or increased pressure. The reaction pressure is, as an absolute pressure, from the viewpoint of the reactivity, preferably 0.09 MPa or more, more preferably 0.10 MPa or more, and is, from the viewpoint of the equipment load, preferably 0.5 MPa or less, more preferably 0.2 MPa or less, even more preferably 0.11 MPa or less. Preferably, the reaction is performed under normal pressure.

<Purification of Oxide>

The oxide production method of the present invention can have a step of further purifying the resultant oxide, after completion of the dehydrogenative oxidation reaction of the organic compound.

In the oxide production method of the present invention, the Pt/Bi composite catalyst can be removed from the reaction liquid containing the oxide of an organic compound after completion of an oxidation reaction of the organic compound, for example, by pressure filtration, or reduced pressure filtration.

In the oxide production method of the present invention, an oxide of an organic compound can be obtained at a high production rate, and therefore, the reaction liquid containing an oxide of the organic compound can be used as it is, as a production raw material for detergents, etc. The organic compound can be obtained at a high concentration by extraction, distillation or the like of the reaction liquid containing an oxide of the organic compounds, as needed.

[Other Production Method for Oxide]

Another production method for an oxide of the present invention is an oxide production method including subjecting an organic compound having one primary hydroxy group to a dehydrogenative oxidation reaction, in the presence of a Pt/Bi composite catalyst, a Bi ion source and water, under the condition such that the minimum value of pH during reaction is less than 7, thereby to obtain an oxide of the organic compound.

According to the other production method for an oxide of the present invention, an oxide of an organic compound can be produced at a high production rate.

In the other production method for an oxide of the present invention, the Pt/Bi composite catalyst is the same as the Pt/Bi composite catalyst obtained in the above-mentioned oxide production method, or as the Pt/Bi composite catalyst obtained in the Pt/Bi catalyst production method to be mentioned below.

The other production method for an oxide of the present invention is preferably accompanied by regeneration of a Pt/Bi composite catalyst.

The reason why the method is preferably accompanied by regeneration of a Pt/Bi composite catalyst is, though not clear, considered as follows.

It is considered that, in repeated use thereof in a production method for an oxide of an organic compound, Bi may gradually dissolve out from the Pt/Bi composite catalyst so that the catalyst structure may change and the catalyst activity may lower.

In the other production method for an oxide of the present invention, it is presumed that, by adding a Bi ion source along with a Pt/Bi composite catalyst, Bi can be reductively precipitated on the Pt/Bi composite catalyst to complement the Bi fraction that has dissolved out and the Pt/Bi composite catalyst can be thereby regenerated. It is also presumed that, along with the regeneration of the Pt/Bi composite catalyst, dehydrogenative oxidation of the organic compound can be promoted so that an oxide of the organic compound can be obtained at a high yield.

From the above, the other production method for an oxide of the present invention has great advantages in that the Pt/Bi composite catalyst can be regenerated in the oxide production process and therefore the method does not require any separate equipment and step for catalyst regeneration and accordingly an oxide of an organic compound can be produced efficiently therefore providing great advantages in point of production efficiency and production cost.

In the oxide production method of the present invention and in the other production method for an oxide thereof, from the viewpoint of efficiently regenerating the Pt/Bi composite catalyst and from the viewpoint of increasing the yield of the oxide of an organic compound, it is preferable to add a Bi ion source at the start of the reaction or during the reaction of dehydrogenative oxidation reaction of the organic compound, and it is more preferable to add a Bi ion source during the reaction.

By adding a Bi ion source to the system at the start of the reaction, the Pt/Bi composite catalyst tends to be prevented from being degraded and the yield of the oxide of an organic compound tends to be increased.

By adding a Bi ion source to the system during the reaction, depending on the degradation state of the Pt/Bi composite catalyst, the Pt/Bi composite catalyst can be regenerated, and therefore the efficiency of the dehydrogenative oxidation of an organic compound can be prevented from lowering, and the yield of the oxide of an organic compound tends to be increased.

Against the problem that Bi dissolves out in recycling use of the catalyst to lower the catalyst activity, there can be provided a method capable of regenerating a high-performance Pt/Bi composite catalyst, without re-preparing of the catalyst.

[Production Method for Pt/Bi Composite Catalyst]

The production method for the Pt/Bi composite catalyst of the present invention is a production method for a Pt/Bi composite catalyst that includes mixing Pt supported by a carrier and a Bi ion source in the presence of an organic compound having one primary hydroxy group and water, and reacting them under the condition such that the minimum value of pH during reaction is less than 7.

According to the production method for a Pt/Bi composite catalyst, a catalyst which exhibits high activity for a dehydrogenative oxidation reaction of an organic compound can be produced.

Though not clear, the reason why the above-mentioned effect can be attained is presumed as follows.

In the case of preparing a catalyst such as a heretofore-known Pt/Bi composite catalyst, in general, a reducing agent is added to a Pt ion source and a Bi ion source under an alkaline condition to precipitate Pt and Bi, thereby preparing a composite catalyst of Pt and Bi. However, it is considered that, in this method, Bi segregates and the resultant composite catalyst has few active structures effective for a dehydrogenative oxidation reaction of an organic compound and the activity of the catalyst is low.

On the other hand, it is presumed that, in the production method for a Pt/Bi composite catalyst of the present invention, Pt supported by a carrier such as a Pt/C catalyst and a Bi ion are kept in contact with each other under the condition such that the minimum value of pH during reaction is less than 7, and accordingly, Bi can be uniformly reductively precipitated on Pt even though a reducing agent is not added, and many active structures effective for a dehydrogenative oxidation reaction of an organic compound can be formed and, as a result, the catalyst of the resultant catalyst can be high.

In the production method for the Pt/Bi composite catalyst of the present invention, from the viewpoint of producing the catalyst having a high activity, preferably, a dehydrogenative oxidation reaction of an organic compound having one primary hydroxy group is accompanied in the reaction system, more preferably a dehydrogenative oxidation reaction of the organic compound is promoted.

Though not clear, the reason why preferably a dehydrogenative oxidation reaction of the organic compound is accompanied, more preferably the reaction is promoted is considered as follows.

By mixing Pt supported by a carrier and a Bi ion source in the presence of the organic compound, a dehydrogenative oxidation reaction of the organic compound is accompanied. Accordingly, a small amount of an oxide of the organic compound is formed to give an oxide-containing solution, and therefore the Bi ion source dissolves to generate a Bi ion, and Bi is reductively precipitated on Pt to produce a Pt/Bi composite catalyst having a higher activity than a Pt catalyst, in the system. It is presumed that a dehydrogenative oxidation reaction of the organic compound is promoted with that, and an oxide of the organic compound can be produced at a high yield.

From the above, preferably a catalyst can be produced at the same time in the production process for the oxide, and therefore not needing any separate equipment and step for catalyst production, an oxide of an organic compound can be produced efficiently, and it is considered that the method of the invention has significant advantages in production efficiency and production cost.

According to the production method for the Pt/Bi composite catalyst of the present invention, it is possible to produce a carboxylic acid compound or a salt of a carboxylic acid compound that is an oxide of the organic compound, along with production of the Pt/Bi composite catalyst.

The carboxylic acid compound or the salt of the carboxylic acid compound include the carboxylic acid compound or a salt of the carboxylic acid compound obtained by subjecting the above-mentioned aliphatic alcohol or polyoxyalkylene alkyl ether to a dehydrogenative oxidation reaction.

Examples of the carboxylic acid compound include a fatty acid to be obtained by subjecting an aliphatic alcohol to a dehydrogenative oxidation reaction and an ether carboxylate to be obtained by subjecting a polyoxyalkylene alkyl ether to a dehydrogenative oxidation reaction.

Examples of the salt of the carboxylic acid compound include neutralized salts of the above-mentioned fatty acid or ether carboxylate with an alkali metal hydroxide where the alkali metal is sodium or potassium.

The organic compound having one primary hydroxy group in the production method for the Pt/Bi composite catalyst of the present invention is preferably an aliphatic alcohol and a polyoxyalkylene alkyl ether, and those exemplified for the organic compound having one primary hydroxy group in the production method for the oxide of the present invention can be used favorably.

The organic compound having one primary hydroxy group is, from the viewpoint of the oxide production rate, preferably an organic compound having high hydrophilicity. The organic compound having one primary hydroxy group is preferably a polyoxyalkylene alkyl ether, more preferably a polyoxyalkylene alkyl ether represented by the above-mentioned general formula (2).

In the production method for the Pt/Bi composite catalyst of the present invention, the reaction can be performed further in the presence of an oxide of the organic compound. The oxide of the organic compound includes a carboxylic acid compound or a salt of a carboxylic acid compound where the hydroxy group that the organic compound has is oxidized, as exemplified in the production method for the oxide of the present invention.

Examples of Pt supported by a carrier that is used in the production method for the Pt/Bi composite catalyst of the present invention can be the same as those of Pt supported by a carrier used in the production method for the oxide of the present invention, and the preferred range thereof is also the same as that of the Pt supported by a carrier used in the production method for the oxide of the present invention.

The carrier for Pt that is supported by a carrier for use in the production method for the Pt/Bi composite catalyst of the present invention is preferably active carbon. Examples of the active carbon can be the same as those of the active carbon for use in the production method for the oxide of the present invention, and the preferred range thereof is also the same as that of the active carbon for use in the production method for the oxide of the present invention.

The Bi ion source in the production method for the Pt/Bi composite catalyst of the present invention is, from the viewpoint of improving the catalyst activity of the Pt/Bi composite catalyst produced, preferably water-insoluble.

Examples of the Bi ion source can be the same as those of the Bi ion source for use in the production method for the oxide of the present invention.

The production method for the Pt/Bi composite catalyst of the present invention is performed in the presence of water. Examples of water can be the same as those of the water for use in the production method for the oxide of the present invention, and the preferred range thereof is also the same as that of the water for use in the production method for the oxide of the present invention.

In the production method for the Pt/Bi composite catalyst of the present invention, the minimum value of pH during reaction is less than 7. By controlling the minimum value of the pH during the reaction to less than 7, the reaction system can be made acidic so that the Bi ion source can dissolve to form a Bi ion, and it is considered that Bi alone from a Bi ion can be prevented from precipitating on the carrier such as active carbon so as to form a structure having a high activity by reductive precipitation of Bi around Pt, and accordingly, reactivity to produce a highly-active Pt/Bi composite catalyst and reactivity for dehydrogenating oxidation reaction of an organic compound can be greatly improved. A preferred range of the pH condition during reaction is the same as that of the pH during reaction in the production method for the oxide of the present invention.

The reaction temperature in the production method for the Pt/Bi composite catalyst of the present invention is the same as the embodiment of the reaction temperature in the oxide production method of the present invention.

In the production method for the Pt/Bi composite catalyst of the present invention, from the viewpoint of subjecting the organic compound to a dehydrogenative oxidation reaction, it is preferable to supply oxygen into the reaction system.

Oxygen supply can be attained by making an oxygen-containing gas flow to run through the liquid phase. Examples of the oxygen-containing gas can be the same as those of the oxygen-containing gas for use in the oxide production method of the present invention, and a preferred range thereof is also the same as that of the oxygen-containing gas for use in the oxide production method of the present invention.

The reaction pressure in the production method for the Pt/Bi composite catalyst of the present invention can be the same as the embodiment of the reaction pressure in the oxide production method of the present invention.

<Recovery of Catalyst>

The catalyst produced in the Pt/Bi composite catalyst production method of the present invention can be collected by separating from the reaction liquid. Examples of the collecting method include increased-pressure filtration and reduced-pressure filtration.

The collected Pt/Bi composite catalyst can be washed, but without being washed, it can be used as a dehydrogenative oxidation reaction catalyst for organic compounds.

The catalyst produced in the Pt/Bi composite catalyst production method of the present invention is, after collected, able to be used again as a dehydrogenative oxidation reaction catalyst for the above-mentioned organic compound.

[Other Production Method for Pt/Bi Composite Catalyst]

Another production method for the Pt/Bi composite catalyst of the present invention is a production method for a Pt/Bi composite catalyst including mixing a Pt/Bi composite catalyst and a Bi ion source in the presence of an organic compound having one primary hydroxy group and water, and reacting them under the condition such that the minimum value of pH during reaction is less than 7.

According to the other production method for the Pt/Bi composite catalyst of the present invention, a catalyst having a high activity for dehydrogenative oxidation reaction of an organic compound can be produced.

In the other production method for the Pt/Bi composite catalyst of the present invention, the Pt/Bi composite catalyst is the same as the Pt/Bi composite catalyst produced according to the above-mentioned Pt/Bi composite catalyst production method of the oxide production method to be mentioned below.

The other production method for the Pt/Bi composite catalyst of the present invention is preferably such that a Bi ion source is added at the start of reaction or during reaction to regenerate a Pt/Bi composite catalyst, and is more preferably such that a Bi ion source is added during reaction to regenerate a Pt/Bi composite catalyst.

By adding a Bi ion source to the system at the start of reaction, the Pt/Bi composite catalyst can be suppressed from being degraded, and the yield of an oxide of an organic compound can be increased.

By adding a Bi ion source to the system during reaction, the Pt/Bi composite catalyst can be regenerated and therefore the efficiency of dehydrogenative oxidation reaction of an organic compound can be suppressed from lowing and the yield of an oxide of an organic compound can be increased.

Though not clear, the reason why regeneration of the Pt/Bi composite catalyst is accompanied by adding a Bi ion source at the start of reaction or during reaction is considered as follows.

It is considered that, in repeated use of the Pt/Bi composite catalyst in the production method for an oxide of an organic compound, Bi gradually dissolves out little by little so that the structure of the catalyst changes and the catalyst activity lowers.

In the other production method for the Pt/Bi composite catalyst of the present invention, it is presumed that, by adding a Bi ion source along with the Pt/Bi composite catalyst, Bi can be reductively precipitated on the Pt/Bi composite catalyst to complement the Bi fraction that has dissolved out and the Pt/Bi composite catalyst can be thereby regenerated. It is also presumed that, along with the regeneration of the Pt/Bi composite catalyst, dehydrogenative oxidation reaction of the organic compound can be promoted so that an oxide of the organic compound can be produced at a high yield.

From the above, the other production method for the Pt/Bi composite catalyst of the present invention has great advantages in that the Pt/Bi composite catalyst can be regenerated in the oxide production process and therefore the method does not require any separate equipment and step for catalyst regeneration and accordingly the Pt/Bi composite can be produced efficiently therefore providing great advantages in point of production efficiency and production cost.

[Oxide of Organic Compound]

The oxide of an organic compound obtained in the oxide production method and the Pt/Bi composite catalyst production method of the present invention can be used in a wide variety of fields as cleaning agents, softeners, wetting agents, dyeing aids and the like. In addition, the oxide is excellent in foaming power and cleaning power, and is further reduced skin irritation, and accordingly, it is favorably used for products that may be in contact with a human skin for a long period of time, such as shampoo, body soap, kitchen detergent, cosmetic composition, and fragrance goods.

According to the oxide production method and the Pt/Bi composite catalyst production method of the present invention, an oxide of an organic compound can be produced at a high yield.

The yield of an oxide of an organic compound in reaction for 24 hours is preferably 75% or more, more preferably 77% or more, even more preferably 80% or more, further more preferably 83% or more.

The yield of an oxide of an organic compound in reaction for 3 hours is preferably 20% or more, more preferably 25% or more, even more preferably 30% or more.

In the present invention, the yield of an oxide of an organic compound can be determined in the same manner as that of the carboxylic acid yield calculation method in Examples given hereinunder.

[Pt/Bi Composite Catalyst]

The Pt/Bi composite catalyst of the present invention can be produced according to the oxide production method and the Pt/Bi composite catalyst production method of the present invention mentioned above.

The Pt/Bi composite catalyst of the present invention is favorably used in reaction of dehydrogenative oxidation of the above-mentioned organic compound having one primary hydroxy group to produce an oxide of the organic compound. Specifically, the present invention also relates to a method of producing an oxide, including dehydrogenative oxidation of an organic compound having one primary hydroxy group to obtain an oxide of the organic compound. The oxide production method of the present invention is preferably dehydrogenative oxidation of an organic compound having one primary hydroxy group, in which oxygen is supplied to a composition containing the organic compound having one primary hydroxy group in the presence of the above-mentioned Pt/Bi composite catalyst and containing water as a solvent.

The amount of Bi supported in the Pt/Bi composite catalyst is, from the viewpoint of improving productivity of the product in oxidation, preferably 0.01% by mass or more, more preferably 0.5% by mass or more, and is preferably 10% by mass or less, more preferably 5% by mass or less, even more preferably 3.5% by mass or less, further more preferably 2.5% by mass or less.

The mass ratio (atomic ratio) of Bi to Pt, Bi/Pt in the Pt/Bi composite catalyst of the present invention is, from the viewpoint of improving the productivity of the product in oxidation reaction, preferably 0.05 or more, more preferably 0.1 or more, and is preferably 2.0 or less, more preferably 1.5 or less, even more preferably 1.0 or less, further more preferably 0.5 or less.

Regarding the above-mentioned embodiments, the present invention further discloses the following oxide production method and Pt/Bi composite catalyst production method.

<1> A production method for an oxide, including subjecting an organic compound having one primary hydroxy group to a dehydrogenative oxidation reaction in the presence of Pt supported by a carrier, a Bi ion source and water under the condition such that the minimum value of the pH during the reaction is less than 7, thereby obtaining an oxide of the organic compound.

<2> A production method for an oxide, including subjecting an organic compound having one primary hydroxy group to a dehydrogenative oxidation reaction in the presence of a Pt/Bi composite catalyst, a Bi ion source and water under the condition such that the minimum value of the pH during the reaction is less than 7, thereby obtaining an oxide of the organic compound.

<3> The production method for an oxide according to <1> or <2>, which is accompanied by preparation of a Pt/Bi composite catalyst.

<4> The production method for an oxide according to any of <1> to <3>, in which the Bi ion source is water-insoluble.

<5> The production method for an oxide according to any of <1> to <4>, in which the Bi ion source is bismuth oxide.

<6> The production method for an oxide according to any of <1> to <5>, in which the charging amount of the Bi ion source is preferably 0.01 parts by mass or more relative to 100 parts by mass of the organic compound, more preferably 0.03 parts by mass or more, even more preferably 0.05 parts by mass or more, and is preferably 1 part by mass or less, more preferably 0.5 parts by mass or less, even more preferably 0.3 parts by mass or less.

<7> The production method for an oxide according to any of <1> to <6>, in which the mass ratio (atomic ratio) of the charging amount of Bi to Pt, Bi/Pt is preferably 0.05 or more, more preferably 0.1 or more, and is preferably 2.0 or less, more preferably 1.8 or less, even more preferably 1.5 or less, further more preferably 1.4 or less, further more preferably 1.2 or less, further more preferably 1.0 or less.

<8> The production method for an oxide according to any of <1> to <7>, in which the organic compound is an aliphatic alcohol or a polyoxyalkylene alkyl ether.

<9> The production method for an oxide according to <8>, in which the aliphatic alcohol or the polyoxyalkylene alkyl ether is preferably one or more represented by the following general formula (1) or general formula (2).

$$R^1O\text{-}H \qquad (1)$$

In the general formula (1), $R^1$ represents a monovalent aliphatic hydrocarbon group having 2 or more and 40 or less carbon atoms.

$$R^2O\text{-}(AO)_n\text{-}H \qquad (2)$$

In the formula (2), $R^2$ represents a monovalent aliphatic hydrocarbon group having 2 or more and 40 or less carbon atoms, A represents an alkanediyl group having 2 or more and 4 or less carbon atoms, AO represents an alkyleneoxy group, n represents an average value of an addition molar number of the alkyleneoxy group, and is 1 or more and 30 or less.

<10> The production method for an oxide according to <9>, in which $R^1$ in the general formula (1) is preferably a linear or branched, primary monovalent aliphatic hydrocarbon group, more preferably a linear or branched, primary alkyl or alkenyl group, even more preferably a linear primary alkyl group.

<11> The production method for an oxide according to <9> or <10>, in which the carbon number of $R^1$ in the general formula (1) is preferably 6 or more, more preferably 8 or more, even more preferably 10 or more, further more preferably 12 or more, and is preferably 36 or less, more preferably 22 or less, even more preferably 18 or less, further more preferably 14 or less.

<12> The production method for an oxide according to any of <9> to <11>, in which $R^2$ in the general formula (2) is preferably a linear or branched, primary aliphatic hydrocarbon group, more preferably a linear or branched, primary alkyl or alkenyl group, even more preferably a linear primary alkyl group.

<13> The production method for an oxide according to any of <9> to <12>, in which the carbon number of $R^2$ in the general formula (2) is preferably 6 or more, more preferably 8 or more, even more preferably 10 or more, further preferably 12 or more, and is preferably 36 or less, more preferably 22 or less, even more preferably 18 or less, further more preferably 14 or less.

<14> The production method for an oxide according to any of <9> to <13>, in which A in the general formula (2) is preferably an ethylene group or a propylene group, more preferably an ethylene group.

<15> The production method for an oxide according to any of <9> to <14>, in which AO in the general formula (2) is preferably an ethyleneoxy group (EO) or a propyleneoxy group (PO), more preferably an ethyleneoxy group(EO).

<16> The production method for an oxide according to any of <9> to <15>, in which n in the general formula (2) is preferably 3 or more, and is preferably 25 or less, more preferably 20 or less, even more preferably 16 or less, further more preferably 12 or less.

<17> The production method for an oxide according to any of <9> to <16>, in which the organic compound is preferably a polyoxyalkylene alkyl ether, more preferably a polyoxyalkylene alkyl ether represented by the general formula (2).

<18> The production method for an oxide according to any of <9> to <16>, in which the oxide of an organic compound is a carboxylic acid compound or a salt of a carboxylic acid compound.

<19> The production method for an oxide according to <18>, in which the carboxylic acid compound or the salt of a carboxylic acid compound is an ether carboxylate or a salt thereof obtained by subjecting a polyoxyalkylene alkyl ether to an oxidation reaction.

<20> The production method for an oxide according to any of <1> to <19>, in which the carrier is active carbon.

<21> The production method for an oxide according to any of <1> to <20>, in which the particle size of Pt supported by the carrier is preferably 1 nm or more, more preferably 3 nm or more, and is preferably 20 nm or less, more preferably 15 nm or less, even more preferably 10 nm or less.

<22> The production method for an oxide according to any of <1> to <21>, in which in the carrier-supported Pt, that is, the amount of Pt supported relative to the total amount of carrier and Pt is preferably 0.1% by mass or more, more preferably 1% by mass or more, even more preferably 2% by mass or more, further more preferably 3% by mass or more, further more preferably 5% by mass or more, and is preferably 20% by mass or less, more preferably 15% by mass or less, even more preferably 10% by mass or less.

<23> The production method for an oxide according to any of <1> to <22>, in which the amount to be charged of Pt supported by a carrier relative to 100 parts by mass of the organic compound is preferably 1 part by mass or more, more preferably 3 parts by mass or more, even more preferably 4 parts by mass or more, and is preferably 10 parts by mass or less, more preferably 8 parts by mass or less, even more preferably 6 parts by mass or less.

<24> The production method for an oxide according to any of <1> to <23>, in which the amount of water relative to 100 parts by mass of the organic compound is preferably 15 parts by mass or more, more preferably 20 parts by mass or more, and is preferably 35 parts by mass or less, more preferably 30 parts by mass or less.

<25> The production method for an oxide according to any of <1> to <24>, in which an alkaline agent and the like is not added to the reaction system.

<26> The production method for an oxide according to any of <1> to <25>, in which the minimum value of pH at the end of the reaction in the oxide production method is preferably 6 or less, more preferably 5 or less, even more preferably 4 or less, and is preferably 1 or more, more preferably 1.5 or more, even more preferably 2 or more.

<27> The production method for an oxide according to any of <1> to <26>, in which the reaction temperature in the oxide production method is preferably 50°C or higher, more preferably 60°C or higher, and is preferably 100°C or lower, more preferably 90°C or lower, even more preferably 85°C or lower.

<28> The production method for an oxide according to any of <1> to <27>, in which in the oxide production method, preferably oxygen is supplied into the reaction system.

<29> The production method for an oxide according to <28>, in which oxygen supply is performed by making an oxygen-containing gas flow to run through the liquid phase.

<30> The production method for an oxide according to <29>, in which the oxygen-containing gas is an oxygen gas or air.

<31> The production method for an oxide according to <30> or <31>, in which the oxygen concentration in the oxygen-containing gas is preferably 10% by volume or more, more preferably 50% by volume or more, even more preferably 70% by volume or more, further more preferably 90% by volume or more, further more preferably 100% by volume.

<32> The production method for an oxide according to any of <1> to <31>, in which in the oxide production method, the reaction pressure is, as an absolute pressure, preferably 0.09 MPa or more, more preferably 0.10 MPa or more, and is preferably 0.5 MPa or less, more preferably 0.2 MPa or less, even more preferably 0.11 MPa or less.

<33> The production method for an oxide according to any of <1> to <32>, in which in the oxide production method, the reaction is preferably performed under normal pressure.

<34> The production method for an oxide according to any of <1> to <33>, in which a Bi ion source is added at the start of the reaction or during the reaction of dehydrogenative oxidation reaction.

<35> The production method for an oxide according to any of <1> to <34>, which further has a step of purifying the resultant oxide after the dehydrogenative oxidation of the organic compound.

<36> The method for producing an oxide according to <35>, in which in the step of purifying the resultant oxide, after the oxidation of the organic compound, the Pt/Bi composite catalyst is removed from the reaction liquid containing the oxide of the organic compound by increased pressure filtration or reduced pressure filtration.

<37> A method for producing a Pt/Bi composite catalyst, including mixing Pt supported by a carrier and a Bi ion source in the presence of an organic compound having one primary hydroxy group and water, and reacting them under the condition such that the minimum value of pH during reaction is less than 7.

<38> A method for producing a Pt/Bi composite catalyst, including mixing a Pt/Bi composite catalyst and a Bi ion source in the presence of an organic compound having one primary hydroxy group and water, and reacting them under the condition such that the minimum value of pH during reaction is less than 7.

<39> The method for producing a Pt/Bi composite catalyst according to <38>, in which a Bi ion source is added at the start of reaction or during reaction to regenerate the Pt/Bi composite catalyst.

<40> The method for producing a Pt/Bi composite catalyst according to <37> to <39>, which is accompanied by dehydrogenative oxidation of the organic compound.

<41> The production method for a Pt/Bi composite catalyst according to any of <37> to <40>, in which the organic compound is an aliphatic alcohol or a polyoxyalkylene alkyl ether.

<42> The production method for a Pt/Bi composite catalyst according to any of <37> to <41>, in which the aliphatic alcohol or the polyoxyalkylene alkyl ether is preferably one or more represented by the following general formula (1) or general formula (2).

$$R^1O\text{-}H \qquad (1)$$

In the general formula (1), $R^1$ represents a monovalent aliphatic hydrocarbon group having 2 or more and 40 or less carbon atoms.

$$R^2O\text{-}(AO)_n\text{-}H \qquad (2)$$

In the formula (2), $R^2$ represents a monovalent aliphatic hydrocarbon group having 2 or more and 40 or less carbon atoms, A represents an alkanediyl group having 2 or more and 4 or less carbon atoms, AO represents an alkyleneoxy group, n represents an average value of an addition molar number of the alkyleneoxy group, and is 1 or more and 30 or less.

<43> The production method for a Pt/Bi composite catalyst according to <42>, in which $R^1$ in the general formula (1) is preferably a linear or branched, primary monovalent aliphatic hydrocarbon group, more preferably a linear or branched, primary alkyl or alkenyl group, even more preferably a linear primary alkyl group.

<44> The production method for a Pt/Bi composite catalyst according to <42> or <43>, in which the carbon number of $R^1$ in the general formula (1) is preferably 6 or more, more preferably 8 or more, even more preferably 10 or more, further more preferably 12 or more, and is preferably 36 or less, more preferably 22 or less, even more preferably 18 or less, further more preferably 14 or less.

<45> The production method for a Pt/Bi composite catalyst according to any of <42> to <44>, in which $R^2$ in the general formula (2) is preferably a linear or branched primary aliphatic hydrocarbon group, more preferably a linear or branched primary alkyl or alkenyl group, even more preferably a linear primary alkyl group.

<46> The production method for a Pt/Bi composite catalyst according to any of <42> to <45>, in which the carbon number of $R^2$ in the general formula (2) is preferably 6 or more, more preferably 8 or more, even more preferably 10 or more, further preferably 12 or more, and is preferably 36 or less, more preferably 22 or less, even more preferably 18 or less, further more preferably 14 or less.

<47> The production method for an oxide according to any of <42> to <46>, in which A in the general formula (2) is preferably an ethylene group or a propylene group, more preferably an ethylene group.

<48> The production method for a Pt/Bi composite catalyst according to any of <42> to <47>, in which AO in the general formula (2) is preferably an ethyleneoxy group (EO) or a propyleneoxy group (PO), more preferably an ethyleneoxy group (EO).

<49> The production method for a Pt/Bi composite catalyst according to any of <42> to <48>, in which n in the general formula (2) is preferably 3 or more, and is preferably 25 or less, more preferably 20 or less, even more preferably 16 or less, further more preferably 12 or less.

<50> The production method for a Pt/Bi composite catalyst according to any of <42> to <49>, in which the organic compound is preferably a polyoxyalkylene alkyl ether, more preferably a polyoxyalkylene alkyl ether represented by the general formula (2).

<51> The production method for a Pt/Bi composite catalyst according to any of <37> to <50>, in which the reaction is performed further in the presence of an oxide of the organic compound.

<52> The production method for a Pt/Bi composite catalyst according to <51>, in which the oxide of an organic compound is a carboxylic acid compound or a salt of a carboxylic acid compound.

<53> The production method for a Pt/Bi composite catalyst according to <52>, in which the carboxylic acid compound or the salt of a carboxylic acid compound is any of a fatty acid obtained by subjecting an aliphatic alcohol to a dehydrogenative oxidation reaction, an ether carboxylate obtained by subjecting a polyoxyalkylene alkyl ether to a dehydrogenative oxidation reaction, and a neutralized salt thereof with an alkali metal hydroxide where the alkali metal is sodium, potassium or the like.

<54> The production method for a Pt/Bi composite catalyst according to any of <37> to <53>, in which the Bi ion source is water-insoluble.

<55> The production method for a Pt/Bi composite catalyst according to any of <37> to <54>, in which the Bi ion source is bismuth oxide.

<56> The production method for a Pt/Bi composite catalyst according to any of <37> to <55>, in which the charging amount of the Bi ion source relative to 100 parts by mass of the organic compound is preferably 0.01 parts by mass or more, more preferably 0.03 parts by mass or more, even more preferably 0.05 parts by mass or more, and is preferably 1 part by mass or less, more preferably 0.5 parts by mass or less, even more preferably 0.3 parts by mass or less.

<57> The production method for a Pt/Bi composite catalyst according to any of <37> to <56>, in which the mass ratio (atomic ratio) of the charging amount of Bi to Pt, Bi/Pt is preferably 0.05 or more, more preferably 0.1 or more, and is preferably 2.0 or less, more preferably 1.8 or less, even more preferably 1.5 or less, further more preferably 1.4 or less, further more preferably 1.2 or less, further more preferably 1.0 or less.

<58> The production method for a Pt/Bi composite catalyst according to any of <37> to <57>, in which the carrier is active carbon.

<59> The production method for a Pt/Bi composite catalyst according to any of <37> to <58>, in which the particle size of Pt supported by the carrier is preferably 1 nm or more, more preferably 3 nm or more, and is preferably 20 nm or less, more preferably 15 nm or less, even more preferably 10 nm or less.

<60> The production method for a Pt/Bi composite catalyst according to any of <37> to <59>, in which in the carrier-supported Pt, that is, the amount of Pt supported relative to the total amount of carrier and Pt is preferably 0.1% by mass or more, more preferably 1% by mass or more, even more preferably 2% by mass or more, further more preferably 3% by mass or more, further more preferably 5% by mass or more, and is preferably 20% by mass or less, more preferably 15% by mass or less, even more preferably 10% by mass or less.

<61> The production method for a Pt/Bi composite catalyst according to any of <37> to <60>, in which the amount to be charged of Pt supported by a carrier is preferably 1 part by mass or more relative to 100 parts by mass of the organic compound, more preferably 3 parts by mass or more, even more preferably 4 parts by mass or more, and is preferably 10 parts by mass or less, more preferably 8 parts by mass or less, even more preferably 6 parts by mass or less.

<62> The production method for a Pt/Bi composite catalyst according to any of <37> to <61>, in which the amount of water is preferably 15 parts by mass or more relative to 100 parts by mass of the organic compound, more preferably 20 parts by mass or more, and is preferably 35 parts by mass or less, more preferably 30 parts by mass or less.

<63> The production method for a Pt/Bi composite catalyst according to any of <37> to <62>, in which the minimum value of pH at the end of the reaction in the Pt/Bi composite catalyst production method is preferably 6 or less, more preferably 5 or less, even more preferably 4 or less, and is preferably 1 or more, more preferably 1.5 or more, even more preferably 2 or more.

<64> The production method for a Pt/Bi composite catalyst according to any of <37> to <63>, in which an alkaline agent and the like is not added to the reaction system.

<65> The production method for a Pt/Bi composite catalyst according to any of <37> to <64>, in which the reaction temperature in the Pt/Bi composite catalyst production method is preferably 50°C or higher, more preferably 60°C or higher, and is preferably 100°C or lower, more preferably 90°C or lower, even more preferably 85°C or lower.

<66> The production method for a Pt/Bi composite catalyst according to any of <37> to <65>, in which in the Pt/Bi composite catalyst production method, preferably oxygen is supplied into the reaction system.

<67> The production method for a Pt/Bi composite catalyst according to <66>, in which oxygen supply is performed by making an oxygen-containing gas flow to run through the liquid phase.

<68> The production method for a Pt/Bi composite catalyst according to <67>, in which the oxygen-containing gas is an oxygen gas or air.

<69> The production method for a Pt/Bi composite catalyst according to <67> or <68>, in which the oxygen concentration in the oxygen-containing gas is preferably 10% by volume or more, more preferably 50% by volume or more, even more preferably 70% by volume or more, further more preferably 90% by volume or more, further more preferably 100% by volume.

<70> The production method for a Pt/Bi composite catalyst according to any of <37> to <69>, in which in the Pt/Bi composite catalyst production method, the reaction pressure is, as an absolute pressure, preferably 0.09 MPa or more, more preferably 0.10 MPa or more, and is preferably 0.5 MPa or less, more preferably 0.2 MPa or less, even more preferably 0.11 MPa or less.

<71> The production method for a Pt/Bi composite catalyst according to any of <37> to <70>, in which in the Pt/Bi composite catalyst production method, the reaction is preferably performed under normal pressure.

<72> A method for producing an oxide, including subjecting an organic compound to a dehydrogenative oxidation reaction in the presence of a Pt/Bi composite catalyst obtained in the Pt/Bi composite catalyst production method of any of <37> to <71> to obtain an oxide of the organic compound.

Examples

Hereinunder the present invention is specifically described with reference to Examples, but the present invention is not whatsoever restricted by these Examples. Physical data were measured and evaluated according to the following methods.

[Production of Pt/Bi Composite Catalyst and Carboxylic Acid Compound]

<Example 1>

Raw materials and the like as mentioned below were charged into a 500-mL five-neck flask equipped with a reflux tube, a dissolved oxygen level meter (by METTLER TOLEDO Corporation), a mechanical stirrer (by IWAKI Corporation, attached with a glass stirring rod fitted with a crescent-type stirring blade (blade width 7.5 cm × height 2.2 cm × thickness 0.4 cm)), glass-made temperature control holder, and a gas-blowing glass tube.

(Charged Raw Materials and the like)

Organic compound: 100 parts by mass (200 g) of AE1 (polyoxyalkylene alkyl ether prepared by adding 3.6 mol on average of ethylene oxide to 1 mol of lauryl alcohol) was charged.

Pt supported by carrier: 5% Pt/C (by Evonik Industries AG, C: charcoal, water content: 59.9% by mass) was charged in an amount, as a solid content excluding the water content, of 4.90 parts by mass (24.45 g).

Bi ion source: 0.11 parts by mass (0.219 g) of bismuth oxide (by FUJIFILM Wako Pure Chemical Corporation) was charged.

Water: Ion-exchanged water was charged so as to be in an amount of 25 parts by mass as combined with the water content of 5% Pt/C (35.35 g).

Next, under nitrogen flow, the above-mentioned five-neck flask was immersed in a water bath, and with stirring the charged raw materials and the like under the condition of 600 rpm, this was heated up to 80°C. After reached 80°C, the nitrogen flow was stopped, and the charged raw materials and the like were bubbled with oxygen at 60 mL/min and reacted under normal pressure for 24 hours.

The reaction was carried out separately every several hours. When suspending the reaction, the reaction temperature was lowered to room temperature, then oxygen bubbling was stopped, nitrogen was circulated, and while the dissolved oxygen level was 0 ppm, the system was kept for 1 hour under the condition and the reaction was stopped. In restarting the reaction, the system was heated up to 80°C with stirring at 600 rpm under nitrogen flow, and then the nitrogen flow was stopped, and the reaction was initiated with bubbling with oxygen at 60 mL/min.

After reaction for 24 hours, oxygen bubbling was stopped, nitrogen flow started, and the condition of the dissolved oxygen level of 0 ppm was kept for 1 hour, and thereafter using a pressure filter, nitrogen was injected under pressure at 4 kgf/cm$^2$ to thereby filter the reaction liquid under pressure to separate the reaction liquid from the catalyst.

During reaction, the reaction liquid was sampled every one hour, and the carboxylic acid compound production rate was determined according to the method mentioned below. The carboxylic acid compound production rate was summarized in Tables 1 to 4.

(Calculation of carboxylic acid compound production rate)

A mixed solution of 60 mL of acetone and 10 mL of ion-exchanged water was added to 0.3 g of the sampled reaction liquid, and stirred to prepare a sample for assay. The sample for assay was titered with an aqueous solution of 0.05 mol/L ethanolic potassium hydroxide to determine the neutralization point. From the thus-determined neutralization point, the experimentally found acid value of the reaction solution was determined, and according to the following calculation formula, the carboxylic acid compound production rate was calculated.

$$\text{Carboxylic acid compound production rate } (\%) = (\text{experimentally found acid value/theoretical acid value}) \times 100$$

Here, the theoretical acid value and the experimentally found acid value were as follows.

(1) Calculation of theoretical acid value: The OHV (hydroxy value) of the organic compound (AE 1) is calculated to be 162 mgKOH/g, and from the molecular weight of the carboxylic acid compound obtained by oxidation of the molecular weight of the organic compound (AE1), the acid value (theoretical acid value) in 100% formation of the carboxylic acid compound by reaction is 156 mgKOH/g.

(2) Calculation of experimentally found acid value: The experimentally found acid value was calculated from the determined neutralization point, according to the following calculation formula.

$$\text{Experimentally found acid value (mgKOH/g)} = \text{titration amount (mL) of aqueous solution of potassium hydroxide} \times 56.11 \text{ (g/mol)} \times 0.05 \text{ (mol/L)/sampling amount (g)/0.7692 (mass ratio of raw material in reaction liquid)}$$

<Examples 2 and 3>

Reaction of Examples 2 and 3 was carried out in the same manner as in Example 1, except that the charging amount of bismuth oxide, as the Bi ion source, was changed as in Table 1. The carboxylic acid compound production rate at 24 hours of reaction is summarized in Table 1.

<Example 4>

Reaction of Example 4 was carried out in the same manner as in Example 1, except that the Bi ion source was changed to 0.23 parts by mass of bismuth nitrate pentoxide (by FUJIFILM Wako Pure Chemical Corporation) as shown in Table 1. The carboxylic acid compound production rate at 24 hours of reaction is summarized in Table 1.

<Example 5>

Reaction of Example 5 was carried out in the same manner as in Example 1, except that the kind of the organic compound was changed to K2098 (Kalcol 2098 (lauryl alcohol), by Kao Corporation) as shown in Table 2. OHV of K2098 is 301 mgKOH/g, and the theoretical acid value of the oxidized product thereof (carboxylic acid compound) is 280 mgKOH/g. The carboxylic acid compound production rate at 7 hours of reaction is summarized in Table 2.

<Example 6>

Reaction of Example 6 was carried out in the same manner as in Example 1, except that the kind of the organic compound was changed to AE2 (polyoxyalkylene alkyl ether produced by adding 9 mol on average of ethylene oxide to 1 mol of 1-octanol) and the reaction time was to 7 hours, as in Table 2. OHV of AE2 is 107 mgKOH/g, and the theoretical acid value of the oxidized product thereof (carboxylic acid compound) is 104 mgKOH/g. The carboxylic acid compound production rate at 7 hours of reaction is summarized in Table 2.

<Comparative Example 1>

Reaction of Comparative Example 1 was carried out in the same manner as in Example 1, except that the Bi ion source was not charged as in Table 1. The carboxylic acid compound production rate at 24 hours of reaction is summarized in Table 1.

<Comparative Example 2>

ApH meter (by Nisshin Rika Co., Ltd.) was attached to a reactor, and while adding an aqueous solution of 48% sodium hydroxide (by Kanto Chemical Co., Inc.) as an alkaline agent so as to control the pH during reaction to be constant at 7.0, reaction of Comparative Example 2 was carried out in the same manner as in Example 1, but the reaction time was 9 hours. The carboxylic acid compound production rate is calculated according to the following method, and the carboxylic acid compound production rate at 3 hours of reaction is summarized in Table 3.

(Calculation of carboxylic acid compound production rate in adding alkaline agent)

0.5 g of 6 M hydrochloric acid (by FUJIFILM Wako Pure Chemical Corporation) was added to 1 g of the sampled reaction liquid, well mixed, and then filtered through a syringe. Subsequently, this was statically left as such for oil/water separation, and 1 mL of methylation reagent (reagent name "TMSI-H", by GL Sciences Inc.) was added thereto, and reacted at 70°C for 10 minutes, then filtered through a syringe, and analyzed by GC (gas chromatography).

(GC measurement condition)

GC Apparatus: Agilent Technologies 6850 (Model Number: Agilent 19091A-102E, by Agilent Technologies Inc.)
Column: Ultra1 Methyl Siloxane (25.0 m $\times$ 200 pm $\times$ 0.33 pm)
Injection temperature 300°C
Detector temperature 300°C
Temperature program:

Kept at 100°C for 5 minutes, and then heated up to 300°C at a rate of 5°C/min (45 minutes in total), and thereafter further kept at 300°C for 40 minutes (program of 90 minutes in total).
Injection volume 1.0 pL
Split ratio 25.0/1
Total flow 28.2 mL/min (gas: He)

<Comparative Example 3>

With adding an aqueous solution of 48% sodium hydroxide (by Kanto Chemical Co., Inc.) as an alkaline agent so as to control the pH during reaction to be constant at 12.0, reaction of Comparative Example 3 was carried out in the same manner as in Comparative Example 2, but the reaction time was 9 hours. The carboxylic acid compound production rate is calculated according to the method described in Comparative Example 2, and the carboxylic acid compound production rate at 3 hours of reaction is summarized in Table 3.

<Reference Example 1>

Reaction of Reference Example 1 was carried out in the same manner as in Example 1, except that the catalyst separated and collected from the reaction liquid after the reaction in Example 1 was charged as Pt supported by a carrier and the Bi ion source, and the reaction time was 17 hours. The sum total of the catalyst collected in Example 1 was 17.80 g. Details of the composition contained in the collected catalyst were, as calculated from the charging amount in Example 1: 10 g of the total of 5% Pt/C and oxide bismuth, 1.56 g of the water content, and 6.24 g of the organic compound (unreacted AE1 and carboxylic acid compound). The carboxylic acid compound production rate at 17 hours of reaction is summarized in Table 4.

Table 1

| | | | Example 1 | Example 2 | Example 3 | Example 4 | Comparative Example 1 |
|---|---|---|---|---|---|---|---|
| Organic Compound | | kind | AE1 | AE1 | AE1 | AE1 | AE1 |
| | | part by mass | 100.00 | 100.00 | 100.00 | 100.00 | 100.00 |
| Pt supported by carrier | 5% Pt/C (solid content in water-containing product) | part by mass | 4.90 | 4.95 | 4.76 | 4.90 | 5.00 |

(continued)

| | | | Example 1 | Example 2 | Example 3 | Example 4 | Comparative Example 1 |
|---|---|---|---|---|---|---|---|
| Bi ion source | $Bi_2O3$ | part by mass | 0.11 | 0.06 | 0.27 | - | - |
| | $Bi(NO_3)_3 \cdot 5H_2O$ | part by mass | - | - | - | 0.23 | - |
| Water | | part by mass | 25.00[*1] | 25.00[*1] | 25.00[*1] | 25.00[*2] | 25.00[*1] |
| Charing amount of Pt atom | | part by mass | 0.245 | 0.248 | 0.238 | 0.245 | 0.250 |
| Charing amount of Bi atom | | part by mass | 0.098 | 0.050 | 0.238 | 0.098 | - |
| Mass ratio of charging amount of Bi atom to Pt atom Bi/Pt | | | 0.400 | 0.200 | 1.000 | 0.400 | - |
| pH at the start of reaction | | | 8.18 | 8.33 | 7.65 | 6.49 | 7.38 |
| pH at the end of reaction | | | 2.44 | 2.30 | 2.52 | 2.39 | 2.84 |
| Carboxylic acid compound production rate at 24 hours of reaction | % | | 90.9 | 84.9 | 82.6 | 77.1 | 74.5 |

*1 Total amount of water content of ion-exchanged water and platinum catalyst-derived water content
*2: Total amount of water content of ion-exchanged water, platinum catalyst-derived water content and bismuth ion source-derived water content

Table 2

| | | | Example 1 | Example 5 | Example 6 |
|---|---|---|---|---|---|
| Organic Compound | | kind | AE1 | K2098 | AE2 |
| | | part by mass | 100.00 | 100.00 | 100.00 |
| Pt supported by carrier | 5% Pt/C (solid content in water-containing product) | part by mass | 4.90 | 4.90 | 4.90 |
| Bi ion source | $Bi_2O3$ | part by mass | 0.11 | 0.11 | 0.11 |
| Water | | part by mass | 25.00[*1] | 25.00[*1] | 25.00[*1] |
| Charing amount of Pt atom | | part by mass | 0.245 | 0.245 | 0.245 |

(continued)

|  | | Example 1 | Example 5 | Example 6 |
|---|---|---|---|---|
| Charing amount of Bi atom | part by mass | 0.098 | 0.098 | 0.098 |
| Mass ratio of charging amount of Bi atom to Pt atom Bi/Pt | | 0.400 | 0.400 | 0.400 |
| pH at the start of reaction | | 8.18 | 7.21 | 7.71 |
| pH at the end of reaction | | 2.44 | 3.92 | 3.00 |
| Carboxylic acid compound production rate at 7 hours of reaction | % | 52.3 | 41.6 | 91.0 |
| *1 Total amount of water content of ion-exchanged water and platinum catalyst-derived water content | | | | |

Table 3

|  | | | Example 1 | Comparative Example 2 | Comparative Example 3 |
|---|---|---|---|---|---|
| Organic compound | | kind | AE1 | AE1 | AE1 |
| | | part by mass | 100.00 | 100.00 | 100.00 |
| Pt supported by carrier | 5% Pt/C (solid content in water-containing product) | part by mass | 4.90 | 4.90 | 4.90 |
| Bi ion source | $Bi_2O3$ | part by mass | 0.11 | 0.11 | 0.11 |
| Alkaline agent | NaOH (effective component in 48% aqueous solution) | part by mass | - | 12.58 | 16.76 |
| Water | | part by mass | 25.00*1 | 31.54*2 | 43.16*2 |
| Charing amount of Pt atom | | part by mass | 0.245 | 0.245 | 0.245 |
| Charing amount of Bi atom | | part by mass | 0.098 | 0.098 | 0.098 |
| Mass ratio of charging amount of Bi atom to Pt atom Bi/Pt | | | 0.400 | 0.400 | 0.400 |
| pH at the start of reaction | | | 8.18 | 8.29 | 12.60 |
| pH at the end of reaction | | | 2.44 | 7.05 | 12.01 |
| Carboxylic acid compound production rate at 3 hours of reaction | | % | 37.9 | 16.7 | 5.4 |
| *1 Total amount of water content of ion-exchanged water and platinum catalyst-derived water content *2: Total amount of water content of ion-exchanged water, platinum catalyst-derived water content and alkaline agent-derived water content | | | | | |

Table 4

|  | | | Example 1 | Reference Example 1 |
|---|---|---|---|---|
| Organic compound | | kind | AE1 | AE1 |
| | | part by mass | 100.00 | 100.00 |

(continued)

|  |  |  | Example 1 | Reference Example 1 |
|---|---|---|---|---|
| Pt supported by carrier | 5% Pt/C (solid content in water-containing product) | part by mass | 4.90 | 8.90*1 |
| Bi ion source | $Bi_2O3$ | part by mass | 0.11 | |
| Water | | part by mass | 25.00*2 | 25.00*3 |
| Charing amount of Pt atom | | part by mass | 0.245 | 0.245 |
| Charing amount of Bi atom | | part by mass | 0.098 | 0.098 |
| Mass ratio of charging amount of Bi atom to Pt atom Bi/Pt | | | 0.400 | 0.400 |
| pH at the start of reaction | | | 8.18 | 4.94 |
| pH at the end of reaction | | | 2.44 | 2.23 |
| Carboxylic acid compound production rate at 17 hours of reaction | | % | 81.6 | 91.3 |
| *1: Total amount (17.80 g) of catalyst collected from Example 1 was charged. (Containing water content 1.56 g and organic compound (including unreacted AE1 and EC) 6.24 g), except catalyst.) *2 Total amount of water content of ion-exchanged water and platinum catalyst-derived water content *3: Total amount of water content of ion-exchanged water, and water content of catalyst collected from Example 1. | | | | |

From Tables 1 to 4, the Pt/Bi composite catalyst obtained by mixing Pt supported by a carrier and a Bi ion source in the presence of an organic compound having one primary hydroxy group and mixing them under the condition such that the minimum value of pH during reaction is less than 7 showed a high carboxylic acid compound production rate at any reaction time, and the intended oxide of an organic compound was obtained efficiently. Also in Reference Example 1 where the catalyst of Example 1 was separated and collected and again used in oxidation reaction of an organic compound, the catalyst exhibited a high carboxylic acid compound production rate.

On the other hand, when the Pt/Bi composite catalyst of Comparative Example 1 where a Bi ion source was not added was used, the carboxylic acid compound production rate at 24 hours of reaction was around 74% and the intended oxide of an organic compound was not obtained efficiently.

Also when the Pt/Bi composite catalyst of Comparative Examples 2 and 3 where an alkaline agent was added during reaction and the minimum value of pH during reaction was not less than 7 was used, the carboxylic acid compound production rate at 3 hours of reaction was 16.7% and 5.4%, respectively, and was low as compared with the carboxylic acid compound production rate at 3 hours of reaction, 37.9% in Example 1. The result is such that the reaction efficiency for an oxidation reaction of an organic compound was poor in these Comparative Examples 2 and 3.

In Reference Example 1, the carboxylic acid compound production rate at 17 hours of reaction was 91.3%, and was higher than the carboxylic acid compound production rate at 17 hours of reaction, 81.6%, in Example 1. This is considered to be because the catalyst of Reference Example 1 uses the catalyst separated and collected from Example 1, that is, in Reference Example 1, since a highly-active Pt-Bi/C catalyst that is presumed to have been already formed in the system was used, there was no time for catalyst formation in the system and an oxidation reaction of the organic compound immediately started after the start of reaction in Reference Example 1.

Industrial Applicability

In the production method for an oxide of the present invention, an oxide of an organic compound can be efficiently produced in the presence of Pt supported by a carrier and a Bi ion source, and further the method can be accompanied by production of a Pt/Bi composite catalyst. Consequently, the method does not require any separate equipment and step for catalyst production and an oxide of an organic compound can be produced efficiently. The resultant oxide of an organic compound can be used in a wide variety of fields as cleaning agents, softeners, wetting agents and dyeing aids.

In the production method for a Pt/Bi composite catalyst of the present invention, a catalyst having high activity for a dehydrogenative oxidation reaction of an organic compound can be produced even in the presence of an organic compound that can be a raw material of an oxide, and further the method can be accompanied by an oxidative dehydrogenation reaction of an organic compound. Consequently, the method does not require any separate equipment and step for catalyst production and an oxide of an organic compound can be produced efficiently, and the resultant Pt/Bi composite catalyst can be favorably used for a reaction to produce an oxide of the organic compound by subjecting an organic compound to a dehydrogenative oxidation reaction.

**Claims**

1. A production method for an oxide, comprising:

   subjecting an organic compound having one primary hydroxy group to a dehydrogenative oxidation reaction in the presence of Pt supported by a carrier, a Bi ion source and water,
   under the condition such that the minimum value of the pH during the reaction is less than 7, thereby obtaining an oxide of the organic compound.

2. The production method for an oxide according to claim 1, which is accompanied by production of a Pt/Bi composite catalyst.

3. The production method for an oxide according to claim 1 or 2, wherein the Bi ion source is water-insoluble.

4. The production method for an oxide according to any of claims 1 to 3, wherein the Bi ion source is bismuth oxide.

5. The production method for an oxide according to any of claims 1 to 4, wherein the organic compound is an aliphatic alcohol or a polyoxyalkylene alkyl ether.

6. The production method for an oxide according to any of claims 1 to 5, wherein the oxide of the organic compound is a carboxylic acid compound or a salt of a carboxylic acid compound.

7. The production method for an oxide according to any of claims 1 to 6, wherein the carrier is active carbon.

8. A production method for an oxide, comprising subjecting an organic compound having one primary hydroxy group to a dehydrogenative oxidation reaction in the presence of a Pt/Bi composite catalyst, a Bi ion source and water, under the condition such that the minimum value of the pH during the reaction is less than 7, thereby obtaining an oxide of the organic compound.

9. The production method for an oxide according to claim 8, which is accompanied by regeneration of the Pt/Bi composite catalyst.

10. The production method for an oxide according to any of claims 1 to 9, wherein a Bi ion source is added at the start or during the reaction of a dehydrogenative oxidation reaction.

11. A production method for a Pt/Bi composite catalyst, comprising:

    in the presence of an organic compound having one primary hydroxy group and water,
    mixing Pt supported by a carrier and a Bi ion source,
    and reacting them under the condition such that the minimum value of the pH during the reaction is less than 7.

12. The production method for a Pt/Bi composite catalyst according to claim 11, which is accompanied by a dehydrogenative oxidation reaction of the organic compound.

13. The production method for a Pt/Bi composite catalyst according to claim 11 or 12, wherein the Bi ion source is water-insoluble.

14. The production method for a Pt/Bi composite catalyst according to any of claims 11 to 13, wherein the Bi ion source is bismuth oxide.

15. The production method for a Pt/Bi composite catalyst according to any of claims 11 to 14, wherein the carrier is active carbon.

16. A production method for a Pt/Bi composite catalyst, comprising:

   in the presence of an organic compound having one primary hydroxy group and water,
   mixing a Pt/Bi composite catalyst and a Bi ion source,
   and reacting them under the condition such that the minimum value of the pH during the reaction is less than 7.

17. The production method for a Pt/Bi composite catalyst according to claim 16, wherein:
   a Bi ion source is added at the start or during the reaction to regenerate the Pt/Bi composite catalyst.

18. A production method for an oxide, comprising subjecting the organic compound to a dehydrogenative oxidation reaction in the presence of a Pt/Bi composite catalyst obtained in the Pt/Bi composite production method of any of claims 11 to 17 to produce an oxide of the organic compound.

## INTERNATIONAL SEARCH REPORT

| International application No. |
|---|
| **PCT/JP2022/012491** |

**A. CLASSIFICATION OF SUBJECT MATTER**

*C07C 51/235*(2006.01)i; *C07C 59/125*(2006.01)i; *C07B 61/00*(2006.01)i; *B01J 37/04*(2006.01)i; *B01J 23/644*(2006.01)i
FI: C07C51/235; B01J37/04 102; C07C59/125 E; C07B61/00 300; B01J23/644 Z

According to International Patent Classification (IPC) or to both national classification and IPC

**B. FIELDS SEARCHED**

Minimum documentation searched (classification system followed by classification symbols)

C07C51/235; C07C59/125; C07B61/00; B01J37/04; B01J23/644

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2012/169585 A1 (KAO CORPORATION) 13 December 2012 (2012-12-13) paragraphs [0001], [0017], [0028]-[0031], [0034], [0036], [0044], [0045], [0060]-[0085], in particular, example 7 | 1-18 |
| Y | | 1-18 |
| X | WO 2016/098801 A1 (KAO CORPORATION) 23 June 2016 (2016-06-23) paragraphs [0001], [0022], [0028], [0033], [0037]-[0039], [0047], [0053]-[0068], [0127]-[0132], [0139], [0140], [0144]-[0149], [0158]-[0165], in particular, example 6 | 11-18 |
| Y | | 1-18 |
| Y | JP 2006-117576 A (TOHO CHEM IND CO LTD) 11 May 2006 (2006-05-11) paragraphs [0001], [0014], [0015], [0017], [0018], in particular, example 2 | 1-18 |
| A | JP 2020-512319 A (BASF SE) 23 April 2020 (2020-04-23) examples 4, 5 | 1-18 |
| A | CN 101816935 A (YOU, Hansheng) 01 September 2010 (2010-09-01) paragraphs [0040]-[0044], [0061]-[0070] | 1-18 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **25 May 2022** | **07 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/012491**

| C. | DOCUMENTS CONSIDERED TO BE RELEVANT |  |
|---|---|---|
| Category* | Citation of document, with indication, where appropriate, of the relevant passages | Relevant to claim No. |
| A | JP 11-140013 A (MITSUI CHEMICALS INC) 25 May 1999 (1999-05-25) paragraphs [0012], [0018] | 1-18 |

Form PCT/ISA/210 (second sheet) (January 2015)

**INTERNATIONAL SEARCH REPORT**
**Information on patent family members**

International application No.

**PCT/JP2022/012491**

| Patent document cited in search report | | | Publication date (day/month/year) | Patent family member(s) | | | Publication date (day/month/year) |
|---|---|---|---|---|---|---|---|
| WO | 2012/169585 | A1 | 13 December 2012 | US 2014/0088323 A1 paragraphs [0001], [0025]-[0029], [0040]-[0048], [0052]-[0054], [0056], [0057], [0066]-[0068], [0113]-[0175] EP 2719678 A1 | | | |
| WO | 2016/098801 | A1 | 23 June 2016 | US 2017/0341070 A1 paragraphs [0001], [0029], [0035], [0040], [0044]-[0046], [0054], [0060]-[0073], [0135]-[0143], [0152], [0153], [0157]-[0162], [0199]-[0209] EP 3235564 A1 | | | |
| JP | 2006-117576 | A | 11 May 2006 | (Family: none) | | | |
| JP | 2020-512319 | A | 23 April 2020 | US 2020/0010393 A1 examples 4, 5 EP 3601205 A1 | | | |
| CN | 101816935 | A | 01 September 2010 | (Family: none) | | | |
| JP | 11-140013 | A | 25 May 1999 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016120484 A
- JP H01146840 A